# EUROPEAN PATENT APPLICATION

(11) **EP 3 130 355 A1**
(43) Date of publication of application: **15.02.2017**
(21) Application number: 15776615.5
(22) Date of filing: 07.04.2015
(51) Int. Cl.: A61K 47/36, A61K 47/10, A61K 47/38, A61K 9/22

(54) **PHARMACEUTICAL COMPOSITION CONTAINING PREGABALIN WITH IMPROVED STABILITY AND METHOD FOR PREPARING SAME**

(30) Priority: 07.04.2014 KR 20140041321
(71) Applicant: Yungjin Pharmaceutical Co., Ltd., Gangdong-gu Seoul 134-721 (KR)
(72) Inventor: AHN, Jae Yong, Yongin-si Gyeonggi-do 446-879 (KR); YOO, Ji Seok, Suwon-si Gyeonggi-do 441-841 (KR); SHIN, Dae-Hee, Seoul 137-860 (KR); RYOO, Byung-Hwan, Seongnam-si Gyeonggi-do 463-845 (KR)
(74) Representative: Smaggasgale, Gillian Helen
(86) International application number: PCT/KR2015/003475
(87) International publication number: WO 2015/156581

(57) **Abstract**

Provided are a sustained-release composition including pregabalin and a pharmaceutically acceptable salt thereof using a gastroretentive drug delivery system (GRDDS), an oral sustained-release formulation using the composition, and a preparation method thereof. In a sustained-release composition according to the present invention and a formulation including the same, a coating compartment including a sugar or a derivative thereof and a plasticizer is introduced onto the outer surface of pregabalin having a less stable structure to ensure stability and to improve compatibility with excipients at the same time, and also to effectively control the release rate. As a result, dosing convenience is improved to provide a gastroretentive drug delivery system having enhanced patient compliance. Therefore, the present invention may exhibit improved therapeutic or prophylactic effects on various neurological diseases, such as neuropathic pain, epilepsy, fibromyalgia syndrome, etc., which have not been easily accomplished due to the characteristics of pregabalin.

## Description

### [Technical Field]

The present disclosure relates to a pharmaceutical composition including pregabalin and a pharmaceutically acceptable salt thereof using a gastroretentive drug delivery system with secured stability, a preparation method thereof, and an oral sustained-release formulation using the composition.

### [Background Art]

Pregabalin, (S)-3-(aminomethyl)-5-methylhexanoic acid (IUPAC Name; (3S-3-(aminomethyl)-5-methylhexanoic acid), refers to a compound known to have a molecular formula of C₈H₁₇NO₂ and a molecular weight of 159.23 and a derivative thereof, and its chemical structure is similar to γ-aminobutyric acid (gamma-aminobutyric acid; GABA), but does not bind to GABA receptors. Pregabalin selectively binds to alpha-2-delta subunits of presynaptic calcium channels in the central nerve system to reduce calcium ion influx at nerve endings. The resulting reduction in calcium influx reduces the release of several excitatory neurotransmitters including glutamate and noradrenalin, leading to restoration of the function of nerve cells to normal levels. Therefore, pregabalin has been developed as a useful drug for the treatment of various neurological diseases such as neuropathic pain, epilepsy, fibromyalgia syndrome, etc., and marketed as an oral immediate release capsule product (under trade name of Lyrica capsule, Pfizer).

Pregabalin is rapidly absorbed in the upper part of the gastrointestinal tract, and reaches peak blood levels within 1.5 hours, and steady-state blood levels are achieved within 24∼48 hours after administration. Further, its half-life is about 6 hours in a normal person. Therefore, the recommended pregabalin dosing range for effective treatment is 150 ∼ 600 mg/day twice or three times a day. Pregabalin may be initiated at a dose of 75 mg, and the dose may be increased gradually. Pregabalin is currently available only as an immediate-release formulation, not a sustained-release (modified release) formulation.

However, administration of twice a day or three times a day which is a clinical way of taking pregabalin causes patients to be considerably uncomfortable, and specifically, greatly decreases a drug compliance of the patients who should take a drug during a long time or a large quantity of drugs altogether. Generally, the administration dose of pregabalin is gradually increased while monitoring reaction of the drug. Therefore, a lack of administration according to reduction in the drug compliance of the patients may lead to aspect completely different from therapeutic effects predicted from the administration dose, thereby generating a great risk of unnecessary increase of the administration dose.

Therefore, by achieving once daily dosing of a sustained-release pregabalin formulation to which a gastroretentive drug delivery system is applied to prolong the gastric retention, drug compliance of elderly patients and patients taking multiple medications may be improved, and undesirable dose-related effects may be prevented by reducing peak blood levels (Cₘₐₓ) and drug efficacy may be also increased by increasing minimum plasma concentrations (Cₘᵢₙ).

The sustained-release formulation of pregabalin, however, presents numerous challenges. First, application of a general sustained release technique is difficult as pregabalin does not exhibit uniform absorption throughout the gastrointestinal tract. Most of pregabalin is absorbed in the upper gastrointestinal tract by L-amino acid transporter, but is poorly absorbed beyond the hepatic flexure. When a general oral sustained release technique is used, the drug released after 6 hours which is an average absorption time of pregabalin travels beyond the hepatic flexure, and therefore, such drug release is clinically ineffective. In order to overcome the disadvantage of the absorption site of pregabalin having an absorption window in the upper part of the small intestine and to ensure its sustained release, there is a need for the development of a formulation using the gastroretentive drug delivery system.

Technologies such as a floating system, an expansion system, a bio-adhesion system, and a high density system have been widely known as the gastroretentive drug delivery system. The floating system is a system to retain a drug in the stomach and intestines for an extended time by using a property which a formulation floats in the gastric juice since the density of the formulation is decreased by a foaming agent. The expansion system is a system to retain a drug in the stomach and intestines for an extended time by expanding the drug with an expandable polymer to prevent the formulation from passing through the pylorus due to size exclusion in the stomach. The bio-adhesion system is a system to retain a drug in the stomach and intestines for an extended time by employing an adhesive polymer to adsorb the drug onto the stomach walls. However, the bio-adhesion system runs short of consistency due to the difference in the amount, viscosity, and metabolic turnover of the viscous fluid between humans, and the drug adsorbed in the gastric mucosa may be drained within an unexpectedly short time over the secretion of the gastric juice. The high density system is a system to retain a formulation in the stomach and intestines for an extended time by increasing the density of the formulation to place the formulation in the antrum part of the stomach. However, it is practically difficult to retain the formulation in the antrum part of the stomach by high density. Of the various systems, the floating system and the expansion system may be practically applicable technologies.

In the application of various technologies, selection of an excipient suitable for the system is essential. Pregabalin, a kind of γ-amino acids, may form PRG-Lactam (S-(+)-4-Isobutyl-pyrrolidin-2-one) by intramolecular cyclization even under normal storage conditions. In the sustained-release formulations, use of various excipients is inevitable, and therefore, it is practically difficult to predict which excipients may cause formation of undesirable related compounds such as PRG-Lactam.

At present, there are many patents regarding sustained-release patterns of pregabalin. For example, Korean Patent Publication No. 2008-0059427 discloses a formulation, which may be swelled at least minimum 9 mm when administrated after a meal or before bed, by using a mixture of polyvinyl acetate and polyvinyl pyrrolidone for the purpose of the control of release to stay pregabalin in stomach as a matrix and using a crosslinked polyvinyl pyrrolidone as a swelling agent. Korean Patent Publication No. 2011-0046360 discloses a sustained-release formulation of pregabalin using a gastroretentive system, in which polyethylene oxide and a polyvinyl alcohol-polyethylene glycol grafted copolymer are used to improve swelling and floating properties of the matrix and to control release of the drug. Korean Patent Publication No. 2011-0123178 discloses a swelling and floating gastroretentive composition including a swelling polymer and a gas generating agent in addition to pregabalin. Korean Patent Publication No. 2013-0023127 discloses a gastroretentive composition further including one or more of a swelling agent, a matrix-forming agent, and a gas generating agent, in addition to pregabalin.

Most technologies of prior arts including these patents have technical features of selectively using particular excipients which are able to improve float and swelling abilities of formulations for longer gastric retention of pregabalin. However, the present inventors observed that direct addition of excipients to pregabalin in the prior arts causes a remarkable increase in related compounds. In other words, due to unstable structural characteristics of pregabalin, use of excipients such as swelling agents or release-controlling agents when the stability is not ensured generates a stability problem.

### [Disclosure]

### [Technical Problem]

Accordingly, the present inventors provide a pregabalin composition having excellent formulation stability by improving its compatibility with excipients. The compatibility with excipients means reciprocal compatibility between pregabalin and respective excipients, and influences of the excipients on pregabalin, such as dosing convenience as well as physical, chemical influences of the excipients. In the composition having secured formulation stability according to the present invention, compatibility with various excipients for the purpose of release control of the drug is improved, and ultimately, the composition is a formulation of which once-a-day administration is possible for the long-term retention of pregabalin in the stomach, and its stability in the pharmaceutical composition may be secured at the same time.

### [Technical Solution]

The present invention is intended to provide a pregabalin composition having excellent formulation stability by improving its compatibility with excipients, the composition eventually being a sustained-release formulation of which once-a-day administration is possible.

To this end, an object of the present invention is to provide a sustained-release pharmaceutical composition with improved stability, which is formulated by forming a coating compartment on the outer surface of a drug compartment including pregabalin and then mixing the drug compartment with a drug release controlling compartment including sustained-release excipients.

Further, another object of the present invention is to provide a preparation method of the sustained-release pharmaceutical composition with improved stability.

Furthermore, still another object of the present invention is to provide a sustained-release pharmaceutical formulation with improved stability, including the sustained-release pharmaceutical composition.

### [Advantageous Effect]

In a sustained-release composition according to the present invention and a formulation including the same, a coating compartment including a sugar or a derivative thereof and a plasticizer is introduced onto the outer surface of pregabalin having a less stable structure to ensure stability and to improve compatibility with excipients at the same time, and also to effectively control the release rate. As a result, dosing convenience is improved (once-a-day formulation) to enhance compliance of a subject to be administered. Therefore, the present invention may exhibit improved therapeutic or prophylactic effects on various neurological diseases, such as neuropathic pain, epilepsy, fibromyalgia syndrome, etc., which have not been easily accomplished due to the characteristics of pregabalin.

### [Brief Description of Drawings]

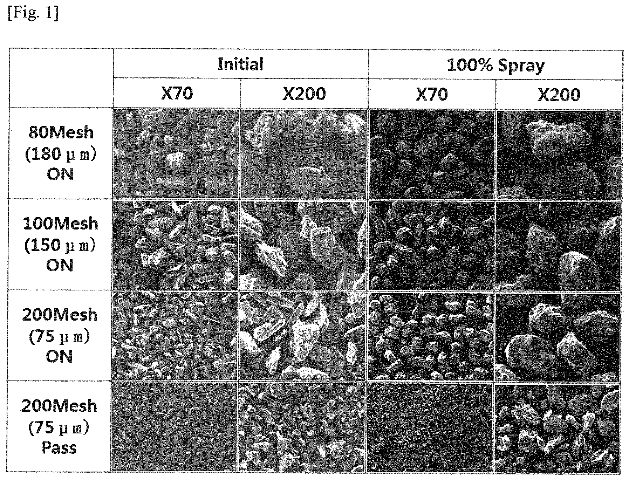

FIG. 1 shows SEM images according to a particle size (80Mesh ON, 100Mesh ON, 200Mesh ON, 200Mesh Pass) of a coating compartment including a main ingredient in a pharmaceutical composition prepared in Preparation Example 1-3; and
FIG. 2 shows the dissolution result of a tablet prepared according to a preparation method of Example 2-2, as measured according to a dissolution method of Experimental Example 1.

### [Best Mode]

The present inventors reproduced and examined a variety of previous technologies regarding sustained-release formulations of pregabalin. As a result, they found that stability is reduced and generation of related compounds is increased by direct addition of various excipients because of poor compatibility between pregabalin and various excipients. Therefore, securing stability of the main ingredient pregabalin is a prerequisite for providing sustained-release formulations of pregabalin. When stability of pregabalin is secured to enhance its compatibility with excipients, a once-a-day formulation for longer retention in the upper part of the gastrointestinal tract may be ultimately achieved, thereby completing the present invention.

The sustained-release pharmaceutical composition of the present invention is formulated by forming a coating compartment on the outer surface of a drug compartment including an active ingredient and then mixing the drug compartment with a drug release controlling compartment including sustained-release excipients.

In a preferred aspect, the present invention relates to a sustained-release pharmaceutical composition with improved stability, the composition including:
(a) a drug compartment including one or two or more active ingredient(s) selected from the group consisting of pregabalin, a pharmaceutically acceptable complex thereof, a salt thereof, a solvate thereof, and a hydrate thereof;
(b) a coating compartment formed on the outer surface of the drug compartment; and
(c) a drug release controlling compartment including pharmaceutically acceptable sustained-release excipients.

In the present invention, pregabalin which is the active ingredient constituting the drug compartment (a) refers to a compound known as (S)-3-(aminnomethyl)-5-methylhexanoic acid, and a derivative thereof. Further, in the present invention, pregabalin may be in any pharmaceutically acceptable form, including a free form thereof, or a pharmaceutically acceptable complex, salt, solvate, hydrate, and polymorph thereof. The term "pharmaceutically acceptable" is employed herein to refer to those materials which may be, within the scope of medical judgment, effectively used for a desired purpose without excessive toxicity, irritation, allergic response, etc. The term "pharmaceutically acceptable salt", as used herein, includes salts derived from pharmaceutically acceptable inorganic acids, organic acids, or bases. Examples of suitable acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzene sulfonic acid, etc. The salts derived from suitable bases may include alkali metals such as sodium, potassium, etc., alkali earth metals such as magnesium, etc., ammonium, etc.

Pregabalin may be chemically prepared by previously known various organic synthetic methods, or may be easily purchased from other commercially available sources. Chemical synthetic methods may be performed with reference to, for example, US Patent No. 5,563,175, US Patent No. 6,046,353, US Patent No. 5,840,956, US Patent No. 5,637,767, US Patent No. 5,629,447, and US Patent No. 5,616,793, but are not limited thereto.

The content of pregabalin in the composition of the present invention may be determined considering a daily dose which exhibits sufficient pharmacological effects without generation of adverse effects and solubility for solvents, and the content may be about 5% by weight to 80% by weight, and preferably about 10% by weight to 60% by weight, based on the total weight of the composition. The general content of pregabalin may be about 50 mg to 900 mg, and preferably 75 mg to 600 mg. If the content of pregabalin is less than 50 mg, sufficient pharmacological effects may not be achieved because of the low concentration of pregabalin, and if the content of pregabalin is more than 900 mg, it is difficult to control sustained-release because of the high concentration of pregabalin.

In the present invention, the coating compartment (a) including pregabalin is characterized in that it is provided as film-coated particles to which a film forming material (coating agent) including a sugar or a derivative thereof is applied in order to ensure stability of pregabalin. The term "particles", as used herein, include granules. Further, the coating compartment may function to improve stability of the drug to enhance its compatibility with various excipients and also to control release of the drug in multi-layers, together with the drug release controlling compartment.

The coating compartment (b) formed on the outer surface of the drug compartment may include a sugar or a derivative thereof as a coating agent.

As described above, although pregabalin is required to use various excipients due to its intrinsic drug absorption characteristic of being mainly absorbed in the upper part of the gastrointestinal tract, various interactions between pregabalin and many excipients generate related compounds, leading to reduction in stability of pregabalin. Consequently, pregabalin has a property of having poor compatibility with excipients. In the present invention, however, the above coating agent, particularly, including a sugar or a derivative thereof is applied to the outer surface of the drug compartment to form the coating compartment, thereby blocking interaction between pregabalin and excipients.

Preferably, the sugar or derivative thereof may be one or more selected from the group consisting of sucrose, mannitol, and sucralose, and more preferably, sucrose or mannitol.

More preferably, the coating compartment may further include a plasticizer. If the plasticizer is included, superior flexibility and adhesion of the plasticizer increase processability so that the sugar or derivative thereof included in the coating agent properly functions as a coating material, and also modify or supplement physical properties of the coating compartment so that improved physical properties of the coating compartment formed after coating are ensured, thereby effectively preventing crack or reduced flexibility of the coating which may occur upon application of the sugar or derivative thereof. Unpredictably, the plasticizer may efficiently function as a stabilizer between raw material molecules of pregabalin to effectively stabilize pregabalin with low excipient compatibility and stability. The plasticizer may be preferably one or more selected from the group consisting of a polyvinyl alcohol-polyethylene glycol grafted copolymer, propylene glycol, diacetin, triacetin, triethyl citrate, diethyl phthalate, polyethylene glycol, dibutyl phthalate, dibutyl sebacate, castor oil, acetylated monoglyceride, and talc.

Of the preferred plasticizers, particularly, the polyvinyl alcohol-polyethylene glycol grafted copolymer is a water soluble polymer having superior flexibility and adhesion, has a linear molecular structure, and is a copolymer of flexible polyethylene glycol and polyvinyl alcohol, and therefore, it may efficiently function as a stabilizer between pregabalin raw materials, and provide processability and flexibility during coating. In particular, the polyvinyl alcohol-polyethylene glycol grafted copolymer is uniformly placed between pregabalin raw materials to decrease compatibility problem with excipients, and functions as a buffer and a lubricant during tabletting, thereby reducing generation of related compounds derived from denaturation due to friction, heat, pressure, etc., which is generated by interaction with excipients and tabletting. A commercially available polyvinyl alcohol-polyethylene glycol grafted copolymer may be exemplified by Kollicoat IR® (BASF), etc.

The sugar or derivative thereof used as the coating agent of the coating compartment which is formed on the outer surface of the drug compartment may be included in an amount of 40% by weight to 98% by weight, preferably 50% by weight to 95% by weight, and the plasticizer may be included in an amount of 2% by weight to 60% by weight, preferably 5% by weight to 50% by weight, on a dry weight basis, based on the total weight of the coating compartment. If the content of the sugar or derivative thereof of the coating compartment is less than 50% by weight, a sufficient coating layer may not be formed, and if the content is more than 95% by weight, flexibility or processability is reduced during formation of the coating compartment of the sugar or derivative thereof, and therefore, a proper coating layer may not be formed. Moreover, if the plasticizer is less than 2% by weight, it does not provide proper flexibility or processability for the coating agent, and therefore, crack may be generated in the formed coating compartment. If the plasticizer is more than 60% by weight, the coating agent becomes too flexible, and therefore, a proper coating layer may not be formed.

In order to form the coating compartment, film-coated drug particles may be obtained by spraying the coating agent while fluidizing the active ingredient. Preferably, film-coated drug particles may be obtained by granulating the coating agent and then powder-coating the outer surface of the active ingredient on a fluidized bed. The particles also include granules.

The content of the coating compartment including the sugar or derivative thereof and the plasticizer, which is formed on the outer surface of the drug compartment, is preferably 10 to 100 parts by weight, based on 100 parts by weight of the active ingredient. Based on 100 parts by weight of the active ingredient, if the coating compartment is less than 10 parts by weight, the stability of the active ingredient may be reduced, and if the coating compartment is more than 100 parts by weight, the size of the formulation may be increased, which causes inconvenience in oral administration.

Further, the sustained-release pharmaceutical composition of the present invention includes a drug release controlling compartment(c) including pharmaceutically acceptable sustained-release excipients.

The sustained-release excipients may include a matrix forming agent, and the matrix forming agent include one or more selected from the group consisting of hydroxypropyl methyl cellulose (HPMC), hydroxyethyl methyl cellulose, hydroxypropyl cellulose, hydroxymethyl cellulose, hydroxyethyl cellulose, carboxymethyl cellulose, calcium carboxymethyl cellulose, sodium carboxymethyl cellulose, methylcellulose, ethylcellulose, polyethylene oxide, locust bean gum, guar gum, xanthan gum, acacia gum, tragacanth gum, alginic acid, sodium alginate, calcium alginate, ammonium alginate, agar, gelatin, poloxamer, polymethacrylate, carbomer, polyvinyl pyrrolidone (PVP), polyvinyl alcohol, polyvinyl acetate (PVAc), polyethylene glycol, a polyvinyl pyrrolidone-polyvinyl acrylate copolymer, a polyvinyl alcohol-polyethylene glycol copolymer, a polyvinyl pyrrolidone-polyvinyl acetate copolymer, bentonite, hectorite, carrageenan, ceratonia, cetostearylalcohol, chitosan, hydroxypropyl starch, magnesium aluminium silicate, polydextrose, poly(methyl vinyl ether/maleic anhydride), polyethylene glycol alginate, and saponite, and preferably, one or more swelling agents selected from the group consisting of hydroxypropyl methylcellulose, polyethylene oxide, carbomer, and poloxamer.

Further, the matrix forming agent may include a gas generating agent, together with the swelling agent. The gas generating agent may be exemplified by sodium carbonate or sodium bicarbonate, but any gas generating agent used in the art may be used without limitation. Sodium bicarbonate is preferred.

Preferably, the sustained-release excipient may further include other pharmaceutically acceptable additives in order to improve the sustained drug release of the active ingredient, in addition to the matrix forming agent. The additives may be exemplified by a binder, a lubricant, a stabilizer, a surfactant, a solubilizer, a sweetening agent, a corrigent, a fragrance, a pigment, a wetting agent, a filler, a thickening agent, a pH adjusting agent, a disintegrating agent, etc., but are not limited thereto, and any pharmaceutically acceptable additives may be used.

For the manufacture of pharmaceutical products, ingredients for a pharmaceutical composition are generally subjected to combining, granulation, drying, milling, spray-coating, blending, lubrication, tabletting, packing, and coating processes. Commonly, an oral unit dosage form is obtained by compression molding (tablet), packing (capsule), or blending (fine granule, drying syrup). To ensure product uniformity and processing flow, ingredients may be combined and managed during processing. For example, the active ingredient may be granulated together with one or more ingredients by granulation, fluidized bed, or extrusion granulation, and then blended with the other ingredients. Similarly, the active ingredient may be first granulated with one or more ingredients constituting the matrix forming agent, and then blended with other excipients, for example, a swelling agent, a gelling agent, a diluent, a lubricant by a subsequent blending process once or more times. To manufacture a final pharmaceutical drug, a compressed dosage form may be subjected to an additional process such as grinding, coating, etc. For discussion of combining, granulation, drying, milling, spray-coating, blending, lubrication, tabletting, packing, coating, etc. and description of technologies of manufacturing pharmaceutical products, a literature [Loyd V. Allen (ed), Remington: The Science and Practice of Pharmacy (Twenty-second edition, 2013)]; a literature [Lieberman, Herbert A. et al. (ed.), Pharmaceutical Dosage Forms: Tablets, Vol. 1-3 (2d ed., 1990)]; and a literature [Dilip M. Parikh et al. (ed), Handbook of Pharmaceutical Granulation Technology, Vol. (Third edition, 2010)]; a literature [Yihong Qiu et al. (ed), Developing Solid Oral Dosage Forms (2009) may be served as references.

Based on these methods of manufacturing pharmaceutical products, in the present invention, the sustained-release excipients included in the drug release controlling compartment may be blended with the swelling agent and the gas generating agent to prepare the matrix forming agent, to which other pharmaceutically acceptable additives may be added. In this regard, the swelling agent, the gas generating agent, and other pharmaceutically acceptable additives may be simply blended to prepare the sustained-release excipient. However, in order to ensure superior stability of pregabalin, it is preferable that other pharmaceutically acceptable additives, or other additives and the gas generating agent may be granulated, and then granules thus prepared may be blended with the swelling agent to prepare the sustained-release excipient. That is, it is preferable that the swelling agent is not included in the granules. If the swelling agent is included in the granules, a formulation is not properly formed in a subsequent formulation molding process, such as tabletting for the formulation of the sustained-release composition according to the present invention.

The drug release controlling compartment may be included in an amount of about 10% by weight to about 50% by weight, preferably about 15% by weight to about 40% by weight, based on the total weight of the composition. If the content of the drug release controlling compartment is less than 15% by weight, a sufficient sustained-release effect may not be obtained, and if the content of the drug release controlling compartment is more than 40% by weight, the active ingredient is not effectively released from the formulation, and therefore, it is difficult to obtain a desired pharmacological effect.

In addition to the constitution of (a)(b)(c), the sustained-release pharmaceutical composition including pregabalin of the present invention may further include other pharmaceutically acceptable excipients for the purpose of facilitating the preparation, compressibility, appearance, and flavor of the formulation.

For example, a stabilizer, a surfactant, a lubricant, a solubilizer, a buffer, a sweetening agent, an adsorbent, a corrigent, a binder, a suspending agent, a hardener, an antioxidant, a polishing agent, a fragrance, a flavoring agent, a pigment, a coating agent, a wetting agent, a moisture adjusting agent, a filler, an antifoaming agent, a refreshing agent, a chewing agent, an antistatic agent, a coloring agent, a sugar coating agent, an isotonic agent, a softening agent, an emulsifying agent, a sticking agent, a thickening agent, a foaming agent, a pH adjusting agent, an excipient, a dispersing agent, a disintegrating agent, a waterproof agent, an antiseptic agent, a preservative, a solubilizing aid, a solvent, a plasticizer, etc. may be added, if needed.

Preferably, the sustained-release pharmaceutical composition of the present invention may further include a diluent, a binder, a disintegrating agent, a lubricant, etc. The diluent may include lactose, celluloses, microcrystalline celluloses, starches, etc., and specifically, lactose may include a lactose monohydrate, a lactose anhydride, a spray dried lactose monohydrate, etc., the microcrystalline celluloses may include microcrystalline cellulose, silicated microcrystalline cellulose, etc., and the starches may include a corn starch, a pregelatinized starch, etc., but are not limited thereto.

The binder may include a polyvinyl alcohol-polyethylene glycol grafted copolymer, polyvinyl pyrrolidone vinyl acetate, ethyl cellulose, hydroxypropyl cellulose, hydroxypropyl methylcellulose, carboxymethylcellulose, methylcellulose, polyvinyl alcohol, polyvinyl pyrrolidone, polyacrylic acid, gelatin, propylene glycol, sodium alginate, etc., but is not limited thereto. The disintegrating agent may include starch, cellulose, cross-linked polymers, e.g., cross-linked polyvinyl pyrrolidone or crospovidone, e.g., POLYPLASDONE XL manufactured by International Specialty Products (USA); cross-linked sodium carboxymethylcellulose or croscarmellose sodium, e.g., AC-D1-SOL manufactured by FMC; and cross-linked calcium carboxymethylcellulose, etc., but is not limited thereto. The lubricant may magnesium stearate, sodium stearyl fumarate, glyceryl behenate, etc., but is not limited thereto. Further, the present invention may include a small amount of pharmaceutically acceptable film coating. The film coating is a water-soluble film-forming material, exemplified by hydroxypropylmethylcellulose (HPMC), hydroxypropylcellulose (HPC), hydroxyethylcellulose (HEC), cellulose acetate phthalate (CAP), ethylcellulose (EC), methylcellulose(MC), polymethacrylate, a polyvinyl alcohol-polyethylene glycol grafted copolymer (Kollicoat®; BASF, Germany), polyvinyl alcohol (PVA) (Opadry (registered trademark); Colorcon, USA), and mixtures thereof, but is not limited thereto.

The sustained-release formulation prepared by including the sustained-release composition of the present invention is not particularly limited to a formulation type, as long as it is an oral formulation. For example, the sustained-release formulation may be formulated into tablets, capsules, granules, fine granules, or pellets, and used for the treatment of various neurological diseases such as neuropathic pain, epilepsy, fibromyalgia syndrome, etc.

Particular administration mode and dosage of the sustained-release formulation of the present invention may be chosen depending on patient's conditions, in particular, age, body weight, lifestyle, and symptom severity, and if necessary, by the physician. Preferably, the sustained-release formulation of the present invention may be provided in a form suitable for once daily administration in order to maintain the plasma concentration of the active ingredient at a constant level and reduce the frequency of administration for patient compliance.

The sustained-release pharmaceutical composition of the present invention is characterized in that it maintains for at least 12 hours, preferably 18 hours, and more preferably 24 hours after oral administration, because pregabalin is released as a sustained-release form for a long time in a desired drug absorption site due to the characteristic constitution of the present invention. According to a specific Experimental Example, it was found that the sustained-release formulation of the present invention was slowly released for effective once daily administration of pregabalin and showed 100% dissolution rate after about 24 hours. In addition, the sustained-release pharmaceutical composition of the present invention is characterized in that it maintains stability without great changes in the dissolution rate under long-term storage conditions (25°C, 60% relative humidity), accelerated conditions (40°C, 75% relative humidity), and severe conditions (60°C, airtight container). Therefore, the composition of the present invention is excellent in terms of taking-easiness and formulation stability.

In another aspect, the present invention relates to a preparation method of the sustained-release pharmaceutical composition including pregabalin or a salt thereof.

Preferably, the preparation method of the present invention includes the steps of:
(a) preparing film-coated drug particles by spraying a coating agent while fluidizing one or two or more active ingredient(s) selected from the group consisting of pregabalin, a pharmaceutically acceptable complex thereof, a salt thereof, a solvate thereof, and a hydrate thereof; and
(b) adding pharmaceutically acceptable sustained-release excipients to the film-coated drug particles, and mixing them with each other.

In the preparation method, forming of the drug coating compartment may be performed by applying the coating agent onto the drug compartment according to a general method known in the pharmaceutical fields. Preferably, the coating agent is sprayed while fluidizing the active ingredients to obtain the film-coated drug particles. More preferably, the coating agent is granulated and applied onto the outer surface of the active ingredients on a fluidized bed to obtain stabilized drug particles.

Further, the sustained-release pharmaceutical composition thus prepared may be prepared as a final formulation according to a general formulation method, for example, a general tablet or capsule preparation method.

### [Detailed Description of the Embodiments]

Hereinafter, the present invention will be described in detail with reference to Examples. However, the following Examples are for illustrative purposes only, and the present invention is not intended to be limited by the following Examples.

### Preparation Example 1-1: Preparation of coating compartment including active ingredient (1)

In order to prepare a coating compartment including an active ingredient (pregabalin), 56 mg of mannitol and 24 mg of Kollicoat IR were first completely dissolved in water to prepare a coating agent. 300 mg of pregabalin was powder-coated with the coating agent to prepare the pregabalin coating compartment with secured stability. In this regard, for powder-coating of pregabalin, a fluidized bed coating machine (Glatt GPCG2 Labsystem, Germany) was used, and the fluidized bed coating machine was operated under conditions of an inlet air temperature of 65°C, a product bed temperature of 30°C, a feeding rate of 1.34 mL/min, a spray nozzle pressure of 1.5 bar, and a spray nozzle diameter of 0.8 mm. The operation conditions of the fluidized bed coating machine are given in Table 1.

**[Table 1]**

| Operation conditions of fluidized bed coating machine | |
|---|---|
| Inlet air Flow | 30 m³/h |
| Inlet air temperature | 65°C |
| Product bed temperature | 30°C |
| Feeding rate | 1.3 mL/min |
| Spray nozzle pressure | 1.5 bar |
| Spray nozzle diameter | 0.8 mm |

The fluidized bed conditions of Table 1 are for the descriptions of the detailed conditions and the preparation method of the present invention, and the scope of the present invention is not intended to be limited thereto.

### Preparation Example 1-2: Preparation of coating compartment including active ingredient (2)

**[Table 2]**

| Raw materials | Content (g) | wt% |
|---|---|---|
| Pregabalin | 300 | 78.9 |
| Sucrose | 56 | 14.7 |
| Kollicoat IR | 24 | 6.3 |
| Purified water (evaporated) | 1,176 | - |
| Total | 380 | 100.0 |

300 g of pregabalin was fluidized in the fluidized bed coating machine under the conditions of Table 1, and then a coating agent was sprayed, the coating agent being prepared by completely dissolving 56 g of sucrose which was 14.7% by weight, based on the total weight of a coating compartment including the main ingredient, and 24 g of Kollicoat IR in 1,176 g of purified water. Thus, the coating compartment including the main ingredient was prepared. The contents of the used ingredients and wt% in the coating compartment thus formed are given as in Table 2.

### Preparation Example 1-3: Preparation of coating compartment including active ingredient (3)

**[Table 3]**

| Raw materials | Content (g) | wt% |
|---|---|---|
| Pregabalin | 300 | 78.9 |
| Mannitol | 56 | 14.7 |
| Kollicoat IR | 24 | 6.3 |
| Purified water (evaporated) | 1,176 | - |
| Total | 380 | 100.0 |

300 g of pregabalin was fluidized in the fluidized bed coating machine under the conditions of Table 1, and then a coating agent was sprayed, the coating agent being prepared by completely dissolving 56 g of mannitol which was 14.7% by weight, based on the total weight of a coating compartment including the main ingredient, and 24 g of Kollicoat IR in 1,176 g of purified water. Thus, the coating compartment including the main ingredient was prepared. The contents of the used ingredients and wt% in the coating compartment thus formed are given as in Table 3.

### Preparation Example 1-4: Preparation of coating compartment including active ingredient (4)

**[Table 4]**

| Raw materials | Content (g) | wt% |
|---|---|---|
| Pregabalin | 300 | 78.9 |
| Sucralose | 56 | 14.7 |
| Kollicoat IR | 24 | 6.3 |
| Purified water (evaporated) | 1,176 | - |
| Total | 380 | 100.0 |

300 g of pregabalin was fluidized in the fluidized bed coating machine under the conditions of Table 1, and then a coating agent was sprayed, the coating agent being prepared by completely dissolving 56 g of sucralose which was 14.7% by weight, based on the total weight of a coating compartment including the main ingredient, and 24 g of Kollicoat IR in 1,176 g of purified water. Thus, the coating compartment including the main ingredient was prepared. The contents of the used ingredients and wt% in the coating compartment thus formed are given as in Table 4.

### Examples 1-1 to 1-4: Preparation of sustained-release composition including pregabalin (1)

According to the composition given in the following Table 5, a sustained-release composition including pregabalin as an active ingredient was prepared. In detail, a coating compartment containing the active ingredient was prepared according to the method described in Preparation Example 1-1, but wt% of each ingredient was as in the following Table 5.

Further, the coating compartment containing the active ingredient thus prepared was mixed with the components in the blending part of Table 5 in a V-Mixer (Dasan pharmatech Co., Ltd. V-mixer120L) at 20 rpm for 10 minutes. Magnesium stearate was added to the mixture thus obtained, followed by post-blending in the V-Mixer (Dasan pharmatech Co., Ltd. V-mixer120L) at 20 rpm for 5 minutes to prepare a final mixture. Tablets were molded using a tablet machine (Kikusui, Hercules2) to a hardness of 10∼15 Kp to prepare plain tablets according to the present invention.

**[Table 5]**

| Components | | Content (mg/tablet) | | | |
|---|---|---|---|---|---|
| | | Example 1-1 | Example 1-2 | Example 1-3 | Example 1-4 |
| Pregabalin -containing coating compartment | Pregabalin | 300 | 300 | 300 | 300 |
| | Mannitol | 75 | 75 | 75 | 61.5 |
| | Kollicoat IR | 31.5 | 31.5 | 31.5 | 18.5 |
| Blending | Microcrystalline cellulosePH102 | 172 | 172 | 172 | 172 |
| | Eudragit RLPO | 30 | 30 | 30 | 30 |
| | HPMC2208 K15M | 300 | 150 | - | 150 |
| | HPMC2208 K100M | - | 150 | 300 | 150 |
| | Sodium bicarbonate | 60 | 60 | 60 | 60 |
| Blending | Magnesium stearate | 8 | 8 | 8 | 8 |
| | Total | 976.5 | 976.5 | 976.5 | 955.0 |

### Examples 2-1 to 2-3: Preparation of sustained-release composition including pregabalin (2)

Plain tablets including the sustained-release composition including pregabalin were prepared in the same manner as in Examples 1-1 to 1-4, except that the composition of the components given in the following Table 6 was used. Separately, 30 mg of Opadry 85F18422 White was dissolved in 351 mg of water, which was used as a coating agent to coat the plain tablets according to a common film coating method. Particle distributions before and after formation of the coating compartment prepared by fluidized bed-coating of the pregabalin are given in Table 7.

**[Table 6]**

| Components | | Content (mg/tablet) | | |
|---|---|---|---|---|
| | | Example 2-1 | Example 2-2 | Example 2-3 |
| Pregabalin- | Pregabalin | 300 | 300 | 300 |
| containing coating compartment | Sucrose | 56 | - | - |
| | Mannitol | - | 56 | - |
| | Sucralose | - | - | 56 |
| | Kollicoat IR | 24 | 24 | 24 |
| Blending | Mannitol | 77 | 77 | 77 |
| | Microcrystalline cellulosePH102 | 165 | 165 | 165 |
| | Eudragit RLPO | 30 | 30 | 30 |
| | HPMC2208 K15M | 150 | 150 | 150 |
| | HPMC2208 K100M | 150 | 150 | 150 |
| | Sodium bicarbonate | 40 | 40 | 40 |
| Post-blending | Magnesium stearate | 8 | 8 | 8 |
| Coating agent | Opadry 85F18422 White | 30 | 30 | 30 |
| | Total | 1,030.0 | 1,030.0 | 1,030.0 |

**[Table 7]**

| Mesh | Size | Initial | 100% Spray |
|---|---|---|---|
| 60Mesh ON | 250µm ON | 60.0% | 92.0% |
| 80Mesh ON | 180µm ON | 12.6% | 5.5% |
| 100Mesh ON | 150µm ON | 9.0% | 2.3% |
| 200Mesh ON | 75µm ON | 12.5% | 0.2% |
| 200Mesh Pass | 75µm Pass | 4.4% | 0.1% |

### Experimental Example 1: Dissolution rate test

A dissolution test was performed according to Dissolution Method of the Korean Pharmacopoeia, 10th edition. A 0.06N-HCl buffer solution was used as a dissolution medium, and the dissolution test was performed using the paddle method under conditions of using 900 mL of the dissolution medium and sinkers at a rotation speed of 50 rpm and a dissolution temperature of 37±0.5°C.

5 mL of a sample was taken at each time point of 0, 1.0, 2.0, 4.0, 6.0, 7.0, 8.0, 10.0, 12.0, 14.0, 16.0, 18.0, 20.0, and 24.0 hrs, respectively. With regard to Examples 1-1 to 1-3, 5 mL of a sample was taken at each time point of 0, 0.5, 1.0, 4.0, 6.0, 12.0, and 24.0 hrs. With regard to Example 1-4, 5 mL of a sample was taken at each time point of 0, 0.5, 1.0, 2.0, 4.0, 6.0, 10.0, and 12.0 hrs. With regard to Examples 2-1 to 2-3, 5 mL of a sample was taken at each time point of 0, 1.0, 2.0, 4.0, 6.0, and 10.0 hrs. Analysis conditions were as follows. The liquid obtained from the dissolution test was filtered using a 0.45 µm membrane filter, and initial 3 ml of the filtrate was discarded, and the next 2 ml of the filtrate was quantified by HPLC. The analysis was performed at a wavelength of 210 nm using a mixture (0.04 M ammonium phosphate ((NH₄)2HPO₄) buffer solution : acetonitrile : methanol = 84 : 5 : 11) containing 5 mM sodium 1-octanesulfonate as a mobile phase, and a flow rate was adjusted (1.5 min/mL) so that a retention time of pregabalin was about 5 minutes. A column was a stainless steel column having an internal diameter of about 4.6 mm and a length of about 250 mm, packed with 5 µm-octadecylsilyl silica gel for liquid chromatography.

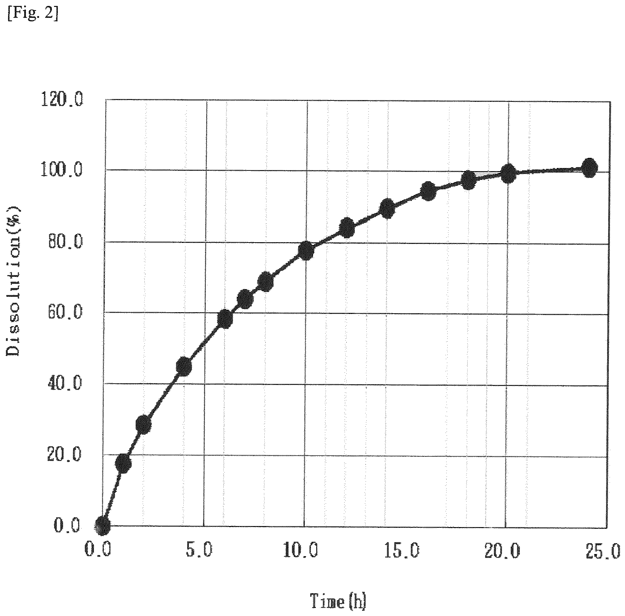

A dissolution graph of FIG. 2 was also obtained from 8 tablets of Example 2-2, which were tested according to the above dissolution method and analysis method. With regard to Example 2-2, samples obtained at each time point of 0, 1.0, 2.0, 4.0, 6.0, 7.0, 8.0, 10.0, 12.0, 14.0, 16.0, 18.0, 20.0, and 24.0 hrs were used to examine the dissolution rate.

**[Table 8]**

| Time | Pregabalin dissolution rate(% w/w) | | | |
|---|---|---|---|---|
| | Example 1-1 | Example 1-2 | Example 1-3 | Example 1-4 |
| 0.0 | 0.0 | 0.0 | 0.0 | 0.0 |
| 0.5 | 6.2 | 5.8 | 6.3 | 7.5 |
| 1.0 | 12.6 | 11.9 | 12.3 | 13.8 |
| 2.0 | | | | 24.2 |
| 4.0 | 35.3 | 35.0 | 34.7 | 38.9 |
| 6.0 | 46.6 | 46.3 | 46.6 | 51.5 |
| 10.0 | | | | 69.2 |
| 12.0 | 74.2 | 73.1 | 73.3 | 76.8 |
| 24.0 | 99.1 | 99.8 | 100.3 | |

**[Table 9]**

| Time (hr) | Pregabalin dissolution rate(% w/w) | | |
|---|---|---|---|
| | Example 2-1 | Example 2-2 | Example 2-3 |
| 0.0 | 0.0 | 0.0 | 0.0 |
| 1.0 | 16.5 | 18.0 | 17.4 |
| 2.0 | 28.1 | 29.0 | 29.0 |
| 4.0 | 45.1 | 46.4 | 47.1 |
| 6.0 | 58.5 | 59.5 | 59.8 |
| 10.0 | 78.3 | 78.8 | 80.9 |
| 12.0 | 86.2 | 88.1 | 89.5 |

### Experimental Example 2: Swellability test

To select a swellable polymer suitable for a gastroretentive formulation, a swelling rate of a tablet including the active ingredient pregabalin and a complex swellable polymer was examined. In detail, Kollidon SR and Plasdone XL, Polyox, HEC, and HPMC were added at a ratio as in the following Table 10, and the swelling rate of the complex swellable tablet including the main ingredient was examined.

As shown in Table 12, the experimental results show that the formulation of Comparative Example 3-1 according to a preparation method disclosed in Korean Patent Publication No. 2008-0059427 (Pfizer) had a size large enough not to pass through the antrum. The swelling rates of Example 3-2 to Example 3-4 were compared with the swelling rate of Comparative Example 3-1 by the images of Table 11. In Table 12, their major axis, minor axis and thickness were examined to confirm their detailed size. 900 mL of 0.06N-HCl buffer solution was used as a dissolution medium, and the dissolution test was performed using the paddle method under conditions of a rotation speed of 50 rpm and a dissolution temperature of 37±0.5°C, and after 4 hours and 8 hours, images and sizes of the tablets were measured using Vernier Calipers (Mitutoyo, Digimatic CD-15).

**[Table 10]**

| Components | | Content (mg/tablet) | | | |
|---|---|---|---|---|---|
| | | Comparative Example 3-1 | Example 3-2 | Example 3-3 | Example 3-4 |
| | Pregabalin | 300.0 | 300.0 | 300.0 | 300.0 |
| | Kollidon SR | 259.0 | 200.0 | | |
| | Plasdone XL | 282.0 | 100.0 | | |
| | Polyox WSR Coagulant | 225.0 | | | |
| | HEC Hx | | 100.0 | | |
| Blending | HPMC2208 K15M | | 300.0 | 150.0 | 130.0 |
| | HPMC2208 K100M | | | | 130.0 |
| | Povidone K-90 | | 60.0 | 80.0 | |
| | Carbopol 71 G | 56.4 | | 50.0 | |
| | Eudragit RLPO | | | | 40.0 |
| | Sodium bicarbonate | | 50.0 | 50.0 | 50.0 |
| | MCC PH-102 | | 200.0 | 200.0 | 200.0 |
| Post-blen ding | Magnesium stearate | 5.6 | 8.0 | 8.0 | 8.0 |
| | Total | 1,128.0 | 1,318.0 | 838.0 | 828.0 |

**[Table 11]**

| | 4h | 8h |
|---|---|---|
| Comparative Example 3-1 | - | 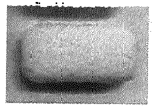 |
| Example 3-2 | 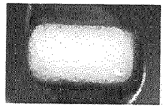 | 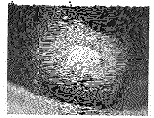 |
| Example 3-3 | 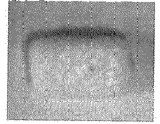 | 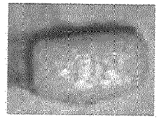 |
| Example 3-4 | 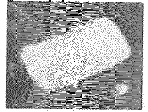 | 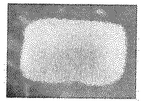 |

**[Table 12]**

| (unit:mm) | 4h | | | 8h | | |
|---|---|---|---|---|---|---|
| | Major axis | Minor axis | Thickness | Major axis | Minor axis | Thickness |
| Comparative Example 3-1 | 23.6 | 12.8 | 12.5 | 23.8 | 13.4 | 13.1 |
| Example 3-2 | 23.2 | 12.3 | 11.5 | 24.0 | 13.0 | 14.8 |
| Example 3-3 | 24.0 | 13.3 | 14.0 | 24.3 | 13.6 | 15.0 |
| Example 3-4 | 23.0 | 12.4 | 11.6 | 23.6 | 12.7 | 14.0 |

### Experimental Example 3: Test of floatability of tablet including pregabalin

To test a floating time of pregabalin with secured stability according to the present invention, each 900 mL was used in 0.06 N HCl heated at 37°C and pH1.2(KP) and the test was performed in a dissolution tester. The time taken for the tablet to float on the surface of the dissolution medium was measured. The results are given in the following Table 13. As shown in Table 13, all the formulations according to the present invention were found to float on the surface of the dissolution medium within 3 minutes to 7 minutes.

**[Table 13]**

| | Time taken to float (min) | |
|---|---|---|
| | 0.06N HCl | pH 1.2 |
| Example 1-1 | 3∼4 | 3∼4 |
| Example 1-2 | 3∼4 | 3∼4 |
| Example 1-3 | 3∼4 | 3∼4 |
| Example 1-4 | 3∼4 | 3∼4 |
| Example 2-1 | 5∼7 | 5∼7 |
| Example 2-2 | 5∼7 | 5∼7 |
| Example 2-3 | 5∼7 | 5∼7 |

### Experimental Example 4: Test of stability of pregabalin according to presence of sugar coating compartment

To evaluate compatibility between pregabalin and excipients and stability of pregabalin in the case of simply mixing pregabalin and the excipients and in the case of forming a coating compartment by coating the outer surface of pregabalin with sugar, the following experiments were performed.

### (1) Stability by simple mixing of pregabalin and excipients

1 g of pregabalin and each 1 g of the excipients given in Table 14 were mixed at room temperature, and the mixture in the form of powder was air-tightened in a glass vial, and this vial was stored under severe conditions (60°C) for 2 weeks, and then a percentage (%) of lactam related compounds (PRG-Lactam) relative to the main peak was calculated by HPLC. The analysis wavelength was 210 nm, a mixture (0.04 M ammonium phosphate ((NH₄)₂HPO₄) buffer solution : acetonitrile : methanol = 84 : 5 : 11) containing 5 mM sodium 1-octanesulfonate was used as a mobile phase A, and acetonitrile was used as a mobile phase B. The mobile phase A was maintained at 100% for initial 6 minutes, and the mobile phase A was maintained from 100% to 70% and the mobile phase B was maintained from 0% to 30% for 6∼45 minutes, and then the mobile phase A was maintained at 70% and the mobile phase B was maintained at 30% for 45∼50 minutes. Post time was 10 minutes. A flow rate was adjusted (0.9 min/mL) so that a retention time of pregabalin was about 8 minutes. A column was a stainless steel column having an internal diameter of about 4.6 mm and a length of about 250 mm, packed with 5 µm-octadecylsilyl silica gel for liquid chromatography. The results are given in the following Table 14.

**[Table 14]**

| | % of Peak area of PRG-Lactam relative to peak area of pregabalin | | |
|---|---|---|---|
| Weeks | 0 | 1 | 2 |
| Pregabalin | 0.074 | 0.196 | 0.329 |
| + Lactose | 0.074 | 0.638 | 0.685 |
| + PEG6000 | 0.072 | 6.206 | 9.839 |
| + Na bicarbonate | 0.076 | 0.204 | 0.348 |
| + Xanthan Gum | 0.114 | 0.427 | 0.936 |
| + Avicel PH102 | 0.038 | 0.422 | 0.944 |
| + PEO | 0.069 | 0.37 | 0.442 |
| + HPMC2208 15K | 0.059 | 1.105 | 2.166 |
| + PVA | 0.046 | 0.292 | 0.515 |
| + Talc | 0.072 | 2.135 | 4.298 |

As shown in Table 14, when pregabalin was simply mixed with many different excipients, a large amount of lactam related compounds were produced over time, and thus stability of pregabalin was remarkably reduced.

### (2) Addition of excipients after sucrose coating of pregabalin

To examine stability of pregabalin when pregabalin is coated with sucrose as a sugar and then excipients are added thereto, 300 g of pregabalin was used, and the preparation method of the coating compartment of Preparation Example 1-2 was performed under the same preparation conditions and method as in Preparation Example 1-1. Thereafter, each of the excipients given in the following Table 15 was physically mixed with the prepared sucrose-coated pregabalin at a weight ratio of 1:1, and then stored for 2 weeks under severe conditions in the same manner as in Experimental Example 4(1). The experimental results are given in the following Table 15.

**[Table 15]**

| | % of Peak area of PRG-Lactam relative to peak area of pregabalin | | |
|---|---|---|---|
| Weeks | 0 | 1 | 2 |
| Sucrose-coated pregabalin | 0.058 | 0.198 | 0.273 |
| + Lactose | 0.079 | 0.253 | 0.332 |
| + PEG6000 | 0.084 | 6.118 | 9.126 |
| + Na bicarbonate | 0.069 | 0.124 | 0.353 |
| + Xanthan Gum | 0.092 | 0.200 | 0.472 |
| + Avicel PH102 | 0.079 | 0.190 | 0.434 |
| + PEO | 0.062 | 0.261 | 0.379 |
| + HPMC2208 15K | 0.075 | 0.307 | 0.398 |
| + PVA | 0.058 | 0.211 | 0.279 |
| + Talc | 0.078 | 0.320 | 0.709 |

As shown in Table 15, when pregabalin was coated with sucrose on its surface and then mixed with excipients, stability of pregabalin was remarkably increased, compared to simple mixing thereof.

### (3) Addition of excipients after mannitol coating of pregabalin

To examine stability of pregabalin when pregabalin is coated with mannitol as a sugar and then excipients are added thereto, 300 g of pregabalin was used, and the preparation method of the coating compartment of Preparation Example 1-3 was performed under the same preparation conditions and method as in Preparation Example 1-1. Thereafter, each of the excipients given in the following Table 16 was physically mixed with the prepared mannitol-coated pregabalin at a weight ratio of 1:1, and then stored for 2 weeks under severe conditions in the same manner as in Experimental Example 4(1). The experimental results are given in the following Table 16.

**[Table 16]**

| | % of Peak area of PRG-Lactam relative to peak area of pregabalin | | |
|---|---|---|---|
| Weeks | 0 | 1 | 2 |
| Mannitol-coated pregabalin | 0.061 | 0.212 | 0.281 |
| + Lactose | 0.077 | 0.248 | 0.319 |
| + PEG6000 | 0.079 | 6.029 | 9.578 |
| + Na bicarbonate | 0.075 | 0.147 | 0.371 |
| + Xanthan Gum | 0.089 | 0.192 | 0.455 |
| + Avicel PH102 | 0.071 | 0.182 | 0.420 |
| + PEO | 0.059 | 0.254 | 0.376 |
| + HPMC2208 15K | 0.070 | 0.282 | 0.372 |
| + PVA | 0.041 | 0.199 | 0.264 |
| + Talc | 0.072 | 0.316 | 0.628 |

As shown in Table 16, when pregabalin was coated with mannitol on its surface and then mixed with excipients, stability of pregabalin was remarkably increased, compared to simple mixing thereof.

### (4) Addition of excipients after sucralose coating of pregabalin

To examine stability of pregabalin when pregabalin is coated with sucralose as a sugar and then excipients are added thereto, 300 g of pregabalin was used, and the preparation method of the coating compartment of Preparation Example 1-4 was performed under the same preparation conditions and method as in Preparation Example 1-1. Thereafter, each of the excipients given in the following Table 17 was physically mixed with the prepared sucralose-coated pregabalin at a weight ratio of 1:1, and then stored for 2 weeks under severe conditions in the same manner as in Experimental Example 4(1). The experimental results are given in the following Table 17.

**[Table 17]**

| | % of Peak area of PRG-Lactam relative to peak area of pregabalin | | |
|---|---|---|---|
| Weeks | 0 | 1 | 2 |
| Sucralose-coated pregabalin | 0.053 | 0.181 | 0.264 |
| + Lactose | 0.059 | 0.217 | 0.298 |
| + PEG6000 | 0.074 | 5.986 | 8.579 |
| + Na bicarbonate | 0.059 | 0.153 | 0.401 |
| + Xanthan Gum | 0.083 | 0.221 | 0.513 |
| + Avicel PH102 | 0.064 | 0.172 | 0.384 |
| + PEO | 0.075 | 0.285 | 0.401 |
| + HPMC2208 15K | 0.057 | 0.251 | 0.352 |
| + PVA | 0.068 | 0.204 | 0.294 |
| + Talc | 0.071 | 0.290 | 0.518 |

As shown in Table 17, when pregabalin was coated with sucralose on its surface and then mixed with excipients, stability of pregabalin was remarkably increased, compared to simple mixing thereof.

## Claims

1. A sustained-release pharmaceutical composition with improved stability, the composition comprising:
(a) a drug compartment comprising one or two or more active ingredient(s) selected from the group consisting of pregabalin, a pharmaceutically acceptable complex thereof, a salt thereof, a solvate thereof, and a hydrate thereof;
(b) a coating compartment formed on the outer surface of the drug compartment; and
(c) a drug release controlling compartment comprising pharmaceutically acceptable sustained-release excipients.

2. The sustained-release pharmaceutical composition with improved stability of claim 1, wherein the coating compartment comprises one or more sugars selected from the group consisting of sucrose, mannitol, and sucralose.

3. The sustained-release pharmaceutical composition with improved stability of claim 2, wherein the coating compartment further comprises a plasticizer.

4. The sustained-release pharmaceutical composition with improved stability of claim 3, wherein the sugar is comprised in an amount of 40% by weight to 98% by weight, and the plasticizer is comprised in an amount of 2% by weight to 60% by weight, on a dry weight basis, based on the total weight of the coating compartment.

5. The sustained-release pharmaceutical composition with improved stability of claim 1, wherein the drug release controlling compartment comprises a matrix forming agent.

6. The sustained-release pharmaceutical composition with improved stability of claim 5, wherein the matrix forming agent comprises one or more swelling agents selected from the group consisting of hydroxypropyl methylcellulose (HPMC), polyethylene oxide, carbomer, and poloxamer.

7. The sustained-release pharmaceutical composition with improved stability of claim 6, wherein the matrix forming agent further comprises one or more gas generating agents selected from the group consisting of sodium carbonate and sodium bicarbonate.

8. The sustained-release pharmaceutical composition with improved stability of claim 5, wherein the matrix forming agent is comprised in an amount of 10% by weight to 50% by weight, based on the total weight of the composition.

9. The sustained-release pharmaceutical composition with improved stability of claim 1, wherein the active ingredient is comprised in an amount of 5% by weight to 80% by weight, based on the total weight of the composition.

10. The sustained-release pharmaceutical composition with improved stability of claim 3, wherein the plasticizer is one or more selected from the group consisting of a polyvinyl alcohol-polyethylene glycol grafted copolymer, propylene glycol, diacetin, triacetin, and triethyl citrate.

11. A preparation method of the sustained-release pharmaceutical composition with improved stability of claim 1, the method comprising the steps of:
(a) preparing drug particles having a film-coated coating compartment by spraying a coating agent while fluidizing one or two or more active ingredient(s) selected from the group consisting of pregabalin, a pharmaceutically acceptable complex thereof, a salt thereof, a solvate thereof, and a hydrate thereof; and
(b) adding pharmaceutically acceptable sustained-release excipients to the film-coated drug particles, and mixing them with each other.

12. The preparation method of claim 11, wherein the coating agent comprises one or more sugars selected from the group consisting of sucrose, mannitol, and sucralose.

13. The preparation method of claim 12, wherein the coating agent further comprises a plasticizer.

14. The preparation method of claim 11, wherein the sustained-release excipients comprise a matrix forming agent.

15. The preparation method of claim 14, wherein the matrix forming agent comprises one or more swelling agents selected from the group consisting of hydroxypropyl methylcellulose (HPMC), polyethylene oxide, carbomer, and poloxamer.

16. The preparation method of claim 15, wherein the matrix forming agent further comprises one or more gas generating agents selected from the group consisting of sodium carbonate and sodium bicarbonate.

17. A sustained-release pharmaceutical formulation with improved stability comprising the sustained-release pharmaceutical composition of claim 1, wherein the sustained-release pharmaceutical formulation is a tablet or a capsule.

18. The sustained-release pharmaceutical formulation with improved stability of claim 17, wherein the formulation further comprises one or more additives selected from the group consisting of a diluent, a binder, a lubricant, a disintegrating agent, and a coating agent.
